Europäisches Patentamt

European Patent Office

Office européen des brevets

(11) Publication number: **0 154 550**
**A2**

## (12) EUROPEAN PATENT APPLICATION

(21) Application number: **85301540.2**

(22) Date of filing: **06.03.85**

(51) Int. Cl.⁴: **C 12 P 21/00**
**C 12 N 15/00, C 12 N 5/00**
**A 61 K 39/395, A 61 K 43/00**
**A 61 K 47/00, G 01 N 33/577**
**A 61 K 49/02**
**//(C12P21/00, C12R1:91)**

(30) Priority: **07.03.84 US 587060**

(43) Date of publication of application:
**11.09.85 Bulletin 85/37**

(84) Designated Contracting States:
**AT BE CH DE FR GB IT LI LU NL SE**

(71) Applicant: **E.I. DU PONT DE NEMOURS AND COMPANY**
**1007 Market Street**
**Wilmington Delaware 19898(US)**

(72) Inventor: **Carney, Walter Patrick**
**15 Anselm Terrace**
**Brighton Massachusetts 02135(US)**

(74) Representative: **Hildyard, Edward Martin et al,**
**Frank B. Dehn & Co. Imperial House 15-19 Kingsway**
**London WC2B 6UZ(GB)**

(54) Monoclonal antibodies capable of binding to pancreatic carcinoma of ductal origin.

(57) Monoclonal antibodies can be obtained which react with neoplastic pancreatic cells of ductal origin, but not with one or more cell types selected from neoplastic pancreatic cells of endocrine origin, regenerative pancreatic cells, pancreatitis cells and normal pancreatic cells. Such antibodies may be tracer-labelled or coupled to a toxic compound or therapeutic radioactive isotope for use in the diagnosis and treatment of primary and metastatic pancreatic cancer.

13E147-149

## Monoclonal antibodies capable of binding to pancreatic carcinoma of ductal origin

This invention concerns monoclonal antibodies that recognize an antigen present on human pancreatic carcinomas. Also of concern are the hybridoma cell lines that secrete the antibodies, as well as tracer-labelled antibodies and antibody fragments, and diagnostic and therapeutic processes that employ the antibodies or antibody fragments of this invention.

The immune response to entry of a foreign substance into the body consists of secretion by plasma cells of "antibodies" which are immunoglobulin (Ig) molecules with combining sites that recognize particular determinants on the surface of the foreign substance, or antigen, and bind to them. Usually, the antibody (Ab) response to an antigen (Ag) is heterogeneous. Upon injection of a body with an immunogen, the body manufactures large numbers of antibodies directed against various components of the antigens and even against various determinant sites on the antigen. It is difficult to separate various antibodies and, thus, conventional antisera contain mixtures of antibodies. It has long been a goal to design a source of antibodies that recognize and combine with specific antigen determinants.

One aspect of hybridoma technology concerns the fusion of myeloma cells with lymphocytes from animals which have been immunized with a particular antigen. Hybridomas manufacture monoclonal antibodies that are specific against a single antigen determinant. Monoclonal antibodies are beginning to replace conventional antisera in standard diagnostic kits for such procedures as radioimmunoassays. Significant work is also being done to adapt hybridoma technology for therapeutic purposes.

Some properties of an ideal fused cell line are: (1) high cloning efficiency; (2) the ability to grow rapidly in a serum medium; (3) no secretion of myeloma Ig; (4) stable production of large amounts of Ig after fusion and (5) ability to grow when reinserted into the originating species. Immunoglobulin is the generic name for various types of antibodies that include IgG, IgM, IgE, and IgD. The various species of Ig have similarities and differences. For example, all molecules of IgG have a constant biological end, sometimes called the leg. However, the immunological ends, sometimes called the arms, will vary according to the antigen determinant which the IgG recognizes.

A typical procedure for making hybridomas is as follows:
(a) immunize mice with a certain immunogen; (b) remove the spleens from the immunized mice and make a spleen suspension in an appropriate medium; (c) fuse the suspended spleen cells with mouse myeloma cells; (d) dilute and culture in separate containers the mixture of unfused spleen cells, unfused myeloma cells and fused cells in a selective medium which will not support growth of the unfused myeloma cells; (e) evaluate the supernatant in each container containing a hybridoma for the presence of antibody to the immunogen; and (f) select and clone hybridomas producing the desired antibodies. Once the desired hybridoma has been selected and cloned, the resultant antibody may be produced by in vitro culturing of the desired hybridoma in a suitable medium. In an alternative method, the desired hybridoma can be injected directly into mice to yield concentrated amounts of antibody.

Hybridomas produced by fusion of murine spleen cells and murine myeloma cells have been described in the literature by Kohler et al., in *Eur. J. Immunol.* 6, 511-519 (1976); by Milstein et al., in *Nature* 266, 550 (1977); and by Walsh, *Nature*, 266, 495 (1977).

The technique is also set out in some detail by Herzenberg and Milstein, in "Handbook on Experimental Immunology", ed. Weir (Blackwell Scientific, London), 1979, pages 25.1 to 25.7. Additional references for hybridoma methodology are provided in "Monoclonal Antibodies" Plenum Press, 1980, R. Kennett, J. McKearn and K. Bectol eds.

Patents relating to monoclonal antibodies against human tumours produced by hybridoma technology include U.S. Patents 4,182,124 and 4,196,265. Representative of the art concerning monoclonal antibodies that have specificity for antigens on carcinoma cells are U.S. patent 4,349,528 and U.S. patent 4,350,683.

In relation to the parent myeloma cell line employed for the fusion event in the example described herein, see Kearney et al, *Immunol.*, 123, 1548-1550 (1978).

Cancer of the pancreas is often a fatal disease and currently ranks fourth among causes of death due to cancer. The major obstacle in diagnosis, management and treatment of pancreatic cancer is the lack of an effective method for early detection. Related publications disclosing monoclonal antibodies to pancreatic tumor cells include Metzgar et al., *Ca Res.*, 42, 601-608 (1982) and Herlyn et al., *J. Clin. Immunol.* 2(2) 135-140 (1982).

According to one aspect of the present invention, we provide a monoclonal antibody which reacts with pancreatic carcinoma of ductal origin, but not with one or more cell types selected from neoplastic pancreatic cells of endocrine origin, regenerative pancreatic cells, pancreatitis cells and normal pancreatic cells. A hybridoma which secretes a monoclonal antibody according to the invention may be prepared by a process which comprises immunizing an animal, for example a mouse, with lung adenocarcinoma cells, for example, A549 lung adenocarcinoma cells, fusing spleen cells from the immunized animal with non-immunoglobulin secreting myeloma cells and selecting a hybridoma which secretes a monoclonal antibody having the desired binding specificity.

The-preparation of the hybridoma cell line 47D10, which secretes a monoclonal antibody according to the invention capable of distinguishing neoplastic pancreatic cells of ductal origin from neoplastic pancreatic cells of endocrine origin, regenerative pancreatic cells, pancreatitis cells and normal pancreatic cells, is described in detail below by way of example. The aforesaid hybridoma cell line was deposited in the ATCC under accession number HB8504 on 24 February 1984.

Balb/c x C57BL/6 mice were immunized with viable A549 lung adenocarcinoma cells derived from bronchioalveolar cancer and mouse spleen cells isolated from an immunized mouse were fused with mouse myeloma cells. The culture supernatants from the resulting hybridomas were screened by enzyme-linked immunosorbent assay (ELISA) and the hybridoma designated 47D10 was selected whose monoclonal antibody exhibited reactivity for the immunogen A549 cells, but not for normal embryonic lung fibroblast cells. After cloning in soft agar, the 47D10 hybridoma was grown in the peritoneal cavity of Balb/c x C57BL/6 mice to produce ascites fluid. The monoclonal antibody (hereinafter referred to as 47D10 antibody) was purified from ascites fluid and evaluated by fixed and live cell ELISA to determine the range of tumor cell reactivity. The antibody was found to be an $IgG_1$ kappa molecule. Normal and neoplastic human tissues were surveyed by immunoperoxidase techniques to determine the immunoreactivity of this monoclonal antibody. In routine paraffin-embedded formalin-fixed material, the antibody demonstrated cell surface and cytoplasmic immunoreactivity in tumour cells of 38 out of 40 pancreatic carcinomas.

Immunizations

Balb/c x C57BL/6 mice were immunized intraperitoneally with $1\times10^7$ viable A549 lung adenocarcinoma cells. Four months later, mouse #1074 was boosted with an intraperitoneal inoculation of $1\times10^7$ viable A549 cells. Spleens were removed for cell fusion 3 days later.

Hybridoma Methodology

Three days after the intraperitoneal boost, the spleen of mouse #1074 was removed and isolated spleen cells were fused with non-secretor myeloma cells of the cell line P3x63Ag8.653, ATCC Accession #CRL 1580, see Kearney et al., Jour. of lmm. 123, 1548-1550 (1979). More particularly, after sacrifice of the mouse, spleen cells suspensions were prepared in serumless DMEM-high glucose medium. Spleen cells from mouse #1074 were mixed with myeloma cells at a ratio of 4:1 in a conical centrifuge tube. This cell mixture was centrifuged at 1200xg for 10 min at room temperature. After removal of the supernatant, the cells were resuspended by tapping the tube. The fusion procedure was initiated by adding 1.0 ml of 50% w/v polyethylene glycol 1000 (Baker) at 37°C over a 30 second period.

The cells were occasionally mixed with a pipette tip for 90 seconds and 5 ml of serumless DMEM-high glucose medium was added over a 3 minute period. This was followed by the addition of 14 ml of DMEM-high glucose medium supplemented with fetal calf serum, non-essential amino acids, L-glutamine, hypoxanthine, aminopterin and thymidine (referred to as HAT medium).

The HAT medium was added over a 1 minute period. Total volume was then adjusted to 50 ml with HAT medium and the cells were centrifuged at 800xg for 10 minutes at room temperature. Supernatants were aspirated and the cell pellet disrupted with 10 ml of HAT medium. $2 \times 10^6$ peritoneal cells (Balb/c x C57BL/6) were added and the final volume adjusted to 50 ml. Cells were then pipetted into 96 well microtiter plates at a final concentration of $2 \times 10^5$ spleen cells per well. Approximately 14 days later, tissue culture supernatants from wells containing hybridoma colonies were tested for binding to methanol-fixed A549 lung adenocarcinoma cells with the enzyme-linked immunosorbent assay (ELISA) procedure. To accomplish this, $5 \times 10^4$ A549 cells were grown in 96 well polyvinyl chloride microtiter plates overnight and fixed to the dishes with 100% methanol. Hybridoma supernatants (0.05 ml) were added to wells containing A549 or normal control cells and incubated overnight at 4°C. Hybridoma 47D10 (ATCC #HB8504) supernatant was selected for fixed cell and live cell ELISA studies, and immunoperoxidase staining cell studies, all discussed below.

Fixed Cell ELISA

Supernatant from hybridoma 47D10 (ATCC #HB8504) was added to wells having normal and tumour cell lines (Table 1) fixed thereto and incubated overnight at 4°C. Hybridoma supernatant was then removed by aspiration and the wells were washed three times with phosphate buffer saline (PBS) containing 1% w/v bovine serum albumin (BSA). Each well subsequently received 0.1 ml of the phosphate buffered saline bovine serum albumin (PBS-BSA) diluted goat anti-mouse IgG antibody conjugated to horseradish peroxidase (GAMHRP) diluted 1:500 in PBS-BSA. Cells were incubated for 60 minutes at 37°C. GAMHRP was removed after incubation and each well washed twice with distilled water. The presence of bound GAMHRP was determined by adding 0.1 ml of the substrate of HRP, 0.2% w/v o-phenylenediamine (OPD) in phosphate buffer (0.009M citric acid, 0.03M $K_2HPO_4$) containing 0.15% hydrogen peroxide. HRP in combination with its substrate results in a yellow coloured product. Development of the product was allowed to occur at room temperature for 15 minutes and the enzymatic reaction was terminated by the addition of 0.1 ml of 4.5 M $H_2SO_4$. Measurement of the resultant reaction product was accomplished by determining optical density (OD) at 488 nm. Presence of yellow colour in the wells indicated that mouse antibody was present in the hybridoma supernatant which could bind an antigen on the cells present and that the mouse antibody could be recognized by the GAMHRP reagent. Hybridoma 47D10 was then cloned in soft agar by standard techniques.

Ascites Production

After 2 agar clonings of hybridoma 47D10, ascites fluid containing the antibody was produced in Balb/c x C57BL/6 mice. Mice were primed with 0.5 ml of Pristane by intraperitoneal (i.p.) injection two weeks prior to inoculation with $2x10^6$ cloned 47D10 hybridoma cells. Two or three weeks later, the ascites fluid was removed and the presence of A549 reactive monoclonal antibody was determined using the ELISA procedure previously described.

Antibody Purification from Ascites Fluid

To the mouse ascites fluid (at 4°C) was added an equivalent amount of saturated ammonium sulphate. This material was stirred at 4°C for 1 hour and the precipitate collected by centrifugation. The precipatate was dissolved in distilled water and dialyzed against 100 volumes of 0.1 M sodium phosphate buffer, pH 8.0, at 4°C. This partially purified

- 8 -

antibody was then purified to a single subclass by protein A-Sepharose chromatography (Pharmacia) according to the procedure of Ey. Prowse and Jenkin, Immunochemistry, 15:429 (1978).

Live Cell ELISA

Normal and tumour cell lines (Table 1) which grow attached to plastic were detached from plastic tissue culture flasks by using standard trypsinization procedures. Cells were seeded in 96 well, flat bottom tissue culture plates at $5 \times 10^4$ viable cells per well and incubated overnight at 37°C in a humidified incubator. After overnight incubation, medium was aspirated and 0.2 ml of a phosphate buffered saline-bovine serum albumin (PBS-BSA) blocking buffer (final concentration of BSA was 5%) was added to each well for a 60 minute incubation at 37°C. Blocking buffer was aspirated and each well washed three times using RPMI-1640 medium containing 1% BSA. Purified 47D10 antibody was added at a concentration of 0.4 µg/ml to each well for a 60 minute incubation. After incubation, the wells were washed with RPMI-1640 medium containing 1% BSA. Each well received 0.1 ml of GAMHRP for 60 minutes at 37°C. The GAMHRP was aspirated, wells were washed three times with RPMI-1640 - 1% BSA, OPD added and colour development measured as previously described for the fixed cell ELISA.

Immunohistochemistry by Immunoperoxidase Staining

Six micron sections of formalin fixed human tissue on slides were used. Fixed tissues were deparaffinized in xylene for 30 minutes at 60°C followed by a 30 minute incubation in xylene at room temperature. Tissues were placed in 100% ethanol followed by incubation with 95% alcohol. In order to block or eliminate endogenous peroxidase activity, tissue sections were incubated for 30 minutes in a 0.2% hydrogen peroxide in methanol mixture.

0154550

Tissue sections were thoroughly rinsed in water followed by washing in PBS. Tissues received 4 drops or normal horse serum for a 30 minute incubation to block nonspecific sticking. The monoclonal antibody secreted by hybridoma 47D10 (ATCC HB8504) was applied and the tissue sections stored overnight at 2°C. After overnight incubation, the tissue sections were washed 3 times in PBS and then incubated for 30 minutes with biotinylated anti-mouse reagents. They were then washed with PBS and incubated for 30 minutes with avidin-biotinylated horseradish peroxidase complex, rinsed in PBS three times and reacted with diaminobenzidine and hydrogen peroxide for 5 minutes. Following this, the tissue sections were rinsed in PBS, counterstained with hematoxylin for 30 seconds and dehydrated. Dehydration was carried out by dipping sections in 80% ethanol for 1 minute, in 95% ethanol for 5 minutes, in 100% ethanol for 5 minutes and in xylene for 10 minutes. Slides were then mounted and examined by microscope to determine the presence of a brownish precipitate which indicates the presence of 47D10 antibody.

ELISA Results

The fixed cell ELISA results for the monoclonal antibody of hybridoma 47D10 (ATCC #HB8504) (summarized in Table 1) show significant antibody binding to the immunogen A549 and the colon cell line WiDr. Studies examining surface binding of the same antibody to live cells (also summarized in Table 1) demonstrated binding to A549, MCF-7, MDA.MB.231, G361 and WiDr cells. Normal fibroblast lines were unreactive. Additional studies with a lung squamous cell carcinoma line, SW900, showed significant binding to both fixed and live tumour cells.

Immunohistochemical Results

To define specificity and range of reactivity, 47D10 monoclonal antibody was evaluated on a variety of human normal and neoplastic tissue sections that were embedded in paraffin. In routine paraffin-embedded formalin-fixed material, the monoclonal antibody demonstrated cell surface and cytoplasmic immunoreactivity with tumour cells of 38 out of 40 pancreatic carcinomas, including pancreatic papillary cystadenocarcinomas as well as both well and poorly differentiated carcinomas of ductal origin (Table II). Pancreatic endocrine tumors were consistently unreactive. Regenerative ductal epithelial seen in chronic pancreatitis as well as normal pancreatic ductal acinar and islet cell constitutents were consistently unreactive. Survey of a variety of normal tissues (lung, pancreas, liver, heart, spleen, kidney, lymph node, bone marrow, cerebral cortex of the brain, skin and adrenal cortex) showed no reactivity. Neoplastic tissues showed immunoreactivity in 5 out of 8 lung and 8 out of 11 colon adenocarcinomas. However, in the case of breast malignancy (frozen sections) this monoclonal antibody showed significant reactivity with infiltrating ductal carcinoma (Table II) with no reactivity with fibrocystic disease of the breast. This monoclonal antibody was also capable of detecting small numbers of metastatic pancreatic tumour cells in lymph nodes by immunoperoxidase staining.

This antibody was also able to distinguish neoplastic pancreatic tissue from regenerative pancreatic ductal structure, from normal tissue and from pancreatitis.

The monoclonal antibody 47D10 and other monoclonal antibodies according to the invention are useful in the diagnosis of primary and metastatic pancreatic tumour cells by conventional in vivo diagnostic methods. Diagnosis may also be carried out by conventional in vitro diagnostic procedures such as the assay of human blood samples or other body fluids. In these procedures, any of the tracer-labels can be utilized (e.g. radiolabels, NMR contrast agents, fluorescents, phosphorescents, chemiluminescents, and bioluminescents). The in situ detection (by gamma scanning) of primary or metastatic pancreatic tumour cells may be effected by coupling the 47D10 monoclonal antibody or another monoclonal antibody according to the invention with appropriate specificity to a radioactive compound by any of the well known techniques, for example, by utilizing a DTPA chelator. Further, the treatment of primary or metastatic pancreatic cancer using the monoclonal antibody 47D10 or another suitable monoclonal antibody according to the invention can be effected by coupling the antibody to a toxic compound or a therapeutic radioactive isotope. Diagnosis may also be carried out by the evaluation of human tissue sections using the same antibodies and the immunohistochemical technique of immunoperoxidase staining. In all the above-mentioned procedures, fragments of monoclonal antibodies according to the invention, comprising an immunological end having an antigen recognition site of appropriate specificity, may also be used. By using appropriate monoclonal antibodies of the present invention or antigen-recognising fragments of such antibodies, the detection of micro-lesions containing only a few tumour cells, that would not ordinarily be detected by conventional staining techniques, is now possible.

- 12 -

## Table 1
## ELISA Analysis

| Cell line tested | Description of Cell Line | Fixed Cell [a] | Live Cells [b] |
|---|---|---|---|
| A549 | Lung adenocarcinoma (ATCC #CCL-185) | ++ | ++ — |
| MCF.7 | Scirrhous carcinoma of the breast (ATCC #HTB-22) | - | + |
| MDA.MB.231 | Intraductal carcinoma of the breast | - | + |
| G361 | Malignant melanoma (ATCC #HTB-26) | + | + |
| SW13 | Adenocarcinoma of the adrenal cortex (ATCC #CCL-105) | - | - |
| WiDr | Adenocarcinoma of the colon (ATCC #CCL-218) | +++ | +++ |
| HEL | Human embryonic lung fibroblast | - | - |
| Flow 5000 | Whole embryo fibroblast | - | - |

(a) Antibody concentration
0.4µg/ml
Optical Density at 488nm
- 0-0.3 OD
+ 0.4-0.9
++ 1.0-1.6
+++ 1.7-2.2

(b) Antibody concentration
10µg/ml
Optical Density at 488nm
- 0-0.3
+ 0.4-0.9
++ 1.0-1.6
+++ 1.7-2.2

0154550

- 13 -

Table II

Immunoperoxidase Staining with 47D10 Monoclonal Antibody

| Tissues tested | #positive or # negative/ # tested |
|---|---|
| 1. Pancreatic adenocarcinoma | 38+/40 |
| 2. Chronic Pancreatitis | 8-/8 |
| 3. Pancreas - islet cell | 1-/1 |
| 4. Pancreas - acute hemorragic necrosis | 1-/1 |
| 5. Breast - infiltrating ductal carcinomas (frozen section) | 4+/7 |
| 6. Breast - fibrocystic disease | 6-/6 |
| 7. Breast - fibrosis | 1-/1 |
| 8. Breast - fibroadenoma | 6-/6 |
| 9. Breast - adenocarcinoma | 1-/1 |
| 10. Lung - squamous cell carcinoma | 3-/3 |
| 11. Lung - oat cell carcinoma | 5-/5 |
| 12. Lung - adenocarcinoma | 5+/8 |
| 13. Lung - adenosquamous | 1-/1 |
| 14. Lung - metastatic | 1+/1 |
| 15. Lung - chronic inflammation | 1-/1 |
| 16. Colon - adenocarcinoma | 8+/11 |
| 17. Colon - adenocarcinoma metatastic to liver | 1-/1 |
| 18. Cecum - adenocarcinoma | 2+/3 |
| 19. Prostate - adenocarcinoma | 1-/1 |
| 20. Overy - benign cystadenoma | 1-/1 |
| 21. Ovary - papillary cystadenoma | 1-/1 |
| 22. Ovary cystadenoma | 1-/2 |
| 23. Common bile duct adenocarcinoma | 6+/6 |
| 24. Bone marrow biopsy (hypercellular - not normal | 2+/2 |

- 14 -

CLAIMS

1.    A monoclonal antibody which reacts with pancreatic carcinoma of ductal origin, but not with pancreatic carcinoma of endocrine origin.

2.    A monoclonal antibody which reacts with pancreatic carcinoma of ductal origin, but not with regenerative pancreatic cells.

3.    A monoclonal antibody which reacts with pancreatic carcinoma of ductal origin, but not with pancreatitis cells.

4.    A monoclonal antibody which reacts with pancreatic carcinoma of ductal origin, but not with normal pancreatic cells.

5.    A monoclonal antibody which reacts with pancreatic carcinoma of ductal origin, but not with normal pancreatic cells, pancreatitis or regenerative pancreatic cells.

6.    A monoclonal antibody as claimed in claim 5 further limited to not exhibiting reactivity with pancreatic carcinoma of endocrine origin.

7.    A monoclonal antibody as claimed in any one of claims 1 to 6 which further reacts with colon adenocarcinoma.

8.    A hybridoma cell line which secreates a monoclonal antibody as claimed in any one of claims 1 to 7.

9.    The hybridoma cell line designated ATCC ≠ HB8504.

10.    The monoclonal antibody secreted by the hybridoma of claim 9.

11.    A fragment of a monoclonal antibody as claimed in any one of claims 1, 2, 3, 4, 5, 6, 7 or 10 comprising an immunological end having an antigen recognition site.

12.    A monoclonal antibody as claimed in any one of claims 1, 2, 3, 4, 5, 6, 7 or 10 further being tracer-labelled.

13.    A monoclonal antibody fragment as claimed in claim 11 further being tracer-labelled.

14.    A monoclonal antibody as claimed in any one
of claims 1, 2, 3, 4, 5, 6, 7, or 10 further being
coupled with a toxic compound or a therapeutic
radioactive isotope.

15.    A monoclonal antibody fragment as claimed
in claim 11 further being coupled with a toxic
compound or a therapeutic radioactive isotope.

16.    A process for the preparation of a hybridoma
cell line as claimed in claim 8 which comprises
immunizing an animal with lung adenocarcinoma cells,
fusing spleen cells from said immunized animal
with non-immunoglobulin secreting myeloma cells
and selecting a hybridoma which secretes a monoclonal
antibody having the desired binding specificity.

17.    A process as claimed in claim 16 wherein
A549 lung adenocarcinoma cells are employed.

18.    A process for the preparation of a monoclonal
antibody as claimed in any one of claims 1 to 7
which comprises culturing a hybridoma cell line
as claimed in claim 8 in vivo and recovering said
antibody from ascites fluid.

19.    A method for in vitro diagnosis of primary
and metastatic pancreatic tumour cells wherein
a monoclonal antibody as claimed in claim 12 or
a monoclonal antibody fragment as claimed in claim
13 is employed.

20.    A monoclonal antibody which reacts with colon
carcinoma, said antibody being secreted by a hybridoma
cell line resulting from the fusion of spleen cells
from a mouse previously immunized with lung adenocarcinoma
and myeloma cells.